(19) Europäisches Patentamt
European Patent Office
Office européen des brevets

(11) Publication number: 0 066 726 B1

(12) EUROPEAN PATENT SPECIFICATION

(45) Date of publication of patent specification: 28.08.85

(51) Int. Cl.[4]: C 12 N 5/00, C 12 M 3/00 // C08B37/02

(21) Application number: 82104142.3

(22) Date of filing: 12.05.82

# (54) Use of a water-swellable and water-insoluble polymeric substance with quaternary amino groups as microcarrier for culturing of anchorage dependant cells.

(30) Priority: 19.05.81 SE 8103138

(43) Date of publication of application:
15.12.82 Bulletin 82/50

(45) Publication of the grant of the patent:
28.08.85 Bulletin 85/35

(84) Designated Contracting States:
DE FR GB NL SE

(56) References cited:
EP-A-0 066 135
FR-A-2 484 419
GB-A-2 043 081
US-A-3 277 025
US-A-3 910 819
US-A-4 189 534

(73) Proprietor: PHARMACIA AB
Rapsgatan 7 Box 604
S-751 25 Uppsala (SE)

(72) Inventor: Carlsson, Mats Erik
Söderby, Akerby
S-740 22 Bälinge (SE)
Inventor: Lindgren, Göran Einar Samuel
Linvreten
S-740 10 Almunge (SE)
Inventor: Gebb-Jones, Christine
Lyckan, Ärentuna
S-755 90 Uppsala (SE)

(74) Representative: Kraus, Walter, Dr. et al
Patentanwälte Kraus Weisert & Partner Thomas-Wimmer-Ring 15
D-8000 München 22 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 066 726 B1

**Description**

The present invention relates primarily to cell biology, in particular, to culturing of anchorage dependent cells on microcarriers *in vitro*.

For a long time there has been a great interest and need for large scale culturing of primary human diploid cells and other anchorage dependent cells. One limiting factor has been the technical difficulties to obtain, by available techniques, an adequate yield of cells or products excreted from the cells. The classical methods for culturing of said cells are culturing in petri dishes, in roller bottles, on different types of fibres, etc. All of these methods have in common the features of the available area being limited, and of a large number of culturing vessels and large volumes of culturing media being needed at scaling up.

Recently an alternative technology for culturing has been developed, which surmount these problems. By allowing the cells to grow on microspheres which altogether offer a very large area in a limited volume, the possibilities for scaling up have been quite altered. AL van Wezel describes in Nature 216 (1967) p 64 a method for culturing of cells on beads of cross-linked dextran having positive substituents. During the introductory work, when the technique using microcarriers was developed, commercially available ion-exchangers of the type DEAE-Sephadex$^R$ (Pharmacia Fine Chemicals) were used. However, for high concentrations of microcarriers toxic effects were observed and the problem of eliminating these were discussed in a number of articles (e.g. by van Wezel in Tissue Culture Methods and Applications, Academic Press, New York 1973 p 372 and by Horng and McLimans in Biotechnol Bioeng 17 (1975) p 713). Levine et al discuss in Somatic Cell Genetics 3 (1977) p 149 the importance of the charge capacity of the microcarriers and they are of the opinion that an optimal product should have a diameter of 150 µm and a capacity of 2 meq/g. The desire for a microcarrier having a charge capacity lower than that of DEAE-Sephadex$^R$ led to developing of Cytodex$^R$ 1 (Pharmacia Fine Chemicals) and Superbeads$^R$ (Flow Laboratories). Both of these may be characterized as low-substituted DEAE-Sephadex$^R$.

The advantages of using beads of cross-linked dextran and other swellable materials are obvious. They have a density (g/cm$^3$) of 1.03—1.045, which in the water-swollen state is negligibly different from the density of the aqueous milieu in which a cell culture is carried out. This is extremely important for culturing of microcarriers, as it is necessary to keep the particles moving without running the risk of tearing the cells off.

Microcarriers synthesized from other organic polymers, such as polystyrene or polyacrylamide, have the drawback of being hydrophobic (water repellant). Moreover they have a density requiring higher stirring speed, which negatively influences the adherence and growth of cells. In order to circumvent this drawback, such microcarriers have been made magnetic which decreases the required stirring speed. The distribution of particles (the particle distribution) should be as narrow as possible so as to achieve a uniform adherence and growth of the cells. Usually a particle size in the range of 150—300 µm is used. It is also of importance that this narrow particle distribution is obtained before the charged substituent is attached. Otherwise a non-uniform charge distribution will be obtained, which might lead to a non-uniform anchorage of the cells.

Moreover the particles should be transparent, which makes microscopying of growing cultures easier.

The uptake of components from the culturing medium, other than those contributing to anchoring and possibly also to growth, should be negligible.

The particles must be non-toxic for primary cells, established cell lines and strains of diploid cells from humans and animals. Moreover, it is necessary that there is no leakage of toxic substances occurring from the microcarrier.

However, in spite of all their advantages, the previously used types of microcarriers have not been found to fulfil all the criteria for a perfect microcarrier being concluded above.

The known microcarriers being based on beads of cross-linked dextran (Superbeads$^R$ and Cytodex$^R$ 1) or polyacrylamide (Biocarriers$^R$ from BIO-RAD Laboratories) contain the substituents

$$—(CH_2)_2—N(C_2H_5)_2$$

or

$$—(CH_2)_3—N(CH_3)_2$$

respectively.

For a long time it has been known that there is a further reaction taking place in the synthesis of polymers with tertiary amines (Ahrgren et al; Polymeric Amines and Ammonium Salts, Ed Goethals, Pergamon Press, Oxford and New York, 1980), which may be illustrated in the following reaction diagram

$$P—O—(CH_2)_2—N(C_2H_5)_2 + Cl(CH_2)_2N(C_2H_5)_2 \rightarrow$$

$$P—O—(CH_2)_2—N^+(C_2H_5)_2—(CH_2)_2—N(C_2H_5)_2$$

where P is the polymer.

It is also known that quarternary amines are degraded at certain conditions (Hoffman degradation). In particular if they contain the so-called beta-hydrogens beta elimination is obtained, which in said three microcarriers results in an alkylating compound which is toxic (C. J. Soper et al; J Pharmacol Suppl (Br Pharm Conf 1979, 31 (1979) p 67).

From US—A—4/189/534 it is already known to use different polymers with quaternary amino groups as cell culture microcarriers. It is also known from GB—A—2 043 081 to prepare microcarriers for cell culture by forming copolymers containing a quaternized tertiary amino group. US—A—3 277 025 teaches appropriate techniques to modify such polymers.

The object of the present invention is to provide better microcarriers for culturing of anchorage dependent cells by using a specific polymeric substance with improved properties.

According to the present invention, this object is achieved by the use of a water-swellable and water-insoluble polymeric substance which has quaternary amino groups of the formula

$$\begin{array}{c} \qquad\qquad\qquad\qquad\qquad\quad R_1 \\ \qquad\qquad\qquad\qquad\qquad\quad | \\ -O-CH_2-CH(OH)-CH_2-N^+-R_3 \\ \qquad\qquad\qquad\qquad\qquad\quad | \\ \qquad\qquad\qquad\qquad\qquad\quad R_2 \end{array}$$

where $R_1$, $R_2$, and $R_3$ which are identical or different, are lower alkyl groups of 1—5 carbon atoms, or lower hydroxyalkyl groups having 2—5 carbon atoms and 1—2 hydroxy groups with at most one hydroxy group being bound to one and the same carbon atom in the hydroxyalkyl group, said quaternary amino groups being located to an outer layer of the microcarrier, which corresponds to 20—30% of the radius of the microcarrier in swollen state, as microcarrier for culturing anchorage dependent cells.

In one preferred embodiment the quarternary amino groups have the formula

$$-O-CH_2-CH(OH)-CH_2-N^+(CH_3)_3.$$

Counter ions to said quarternary amino groups may be inorganic or organic negative ions, which are not toxic for the cells, e.g. chloride ions or sulphate ions, or negative ions of non-toxic organic acids.

Those polymeric matrices being known from chromatography are advantageously used as carriers. Examples thereof are crosslinked dextran, agarose etc.

The previously mentioned advantageous properties of crosslinked dextran make it particularly suitable as a carrier. The synthesis of polymeric matrices is well-known. Moreover, it is well-known that they may be obtained in form of small spherical particles by the so-called bead-polymerization.

According to the invention, it is preferable that the total charge capacity of the microcarriers is in the range of 0.3—2.0 meq/g by product (with chloride as a counter ion).

Microcarriers according to the invention may be prepared by known technique, analogously to that described in US—A—3 277 025.

Moreover, it has, according to the invention, been shown that an optimum of cell yield is achieved at a total charge capacity of 0.5—0.8 meq/g dry product (with chloride as a counter ion).

Thereby, microcarriers of different charge may obtain substantially the same charge density calculated per volume unit in the substituted layer. It is of importance that the substituted layer occupies a certain part of the radius. By localizing the charge to the surface a further advantage is a considerable reduction in the adsorption of components either added to or excreted by the cells by ion exchange.

According to one embodiment of the invention, the localization of the charge to the surface of microcarriers is accomplished by reacting the waterswellable microcarriers, in the presence of a suitable solvent and therefore in an unswollen state, with a compound in order to introduce the group:

$$\begin{array}{c} \qquad\qquad\qquad\qquad\qquad\quad R_1 \\ \qquad\qquad\qquad\qquad\qquad\quad | \\ -O-CH_2-CH(OH)-CH_2-N^+-R_2 \\ \qquad\qquad\qquad\qquad\qquad\quad | \\ \qquad\qquad\qquad\qquad\qquad\quad R_3 \end{array}$$

Where $R_1$, $R_2$ and $R_3$ have the meanings given above.

The invention will be further illustrated by the following working examples.

Example 1

5 g of cross-linked beaded dextran (type Sephadex[R] G-50, Pharmacia Fine Chemicals AB, Uppsala, Sweden) of bead size 60—87 μm was suspended in 10 ml 95% ethanol in a vessel of 50 ml equipped with a stirrer and a refluxer. Thereafter 12 g glycidyl trimethyl ammonium chloride was added and the mixture was heated to 60°C followed by addition of 7.5 ml triethylamine. After 6 hours the mixture was cooled to room temperature and suspended in 5×150 ml 0.9% sodium chloride solution. The yield was 5 g of a surface-substituted bead of cross-linked dextran having a total capacity of 0.60 meq/g.

About 10 g of this dry microcarrier was swollen in water on a glass filter. 3 drops of a 10% sodium hydroxide solution were added and the mixture was washed in distilled water to neutral reaction. 2 mg FITC dextran was dissolved in 2 ml water and added. FITC dextran is a soluble dextran of molecular weight 10 000 to which fluorescein isothiocyanate is covalently bound as a ligand. The mixture was equillibrated for 10 minutes and was then washed with water. This coloured microcarrier was placed on an object glass and examined in a fluorescence microscope. Thereby it was established that the charge was localized to a layer corresponding to 20% of the radius of the microcarrier in the swollen state.

Example 2

$1\times10^5$ VERO cells/ml (VERO cells are an established cell line from the kidney of African green monkey, and are commercially available from Flow Laboratories Ltd., England) were incubated with 2 g/l of autoclaved microcarrier in growth medium (Dulbecco's modified Eagle's medium containing 4 mM glutamine and 10% foetal calf serum from Flow Laboratories) at 37°C

with 5% $CO_2$ and a humidity of 95%. As a microcarrier those prepared according to example 1 and others of different total capacities synthesized in the same way were used. The cells were counted according to Sanford et al (J Natl Cancer Inst *11*, 737, (1951) after 55 hours in culture.

| Capacity meq/g | Cells per ml |
|---|---|
| 0.16 | $4.2\times10^4$ |
| 0.51 | $9.2\times10^4$ |
| 0.60 | $1.0\times10^5$ |
| 0.74 | $1.0\times10^5$ |
| 0.89 | $8.2\times10^4$ |

Example 3

$10^5$ VERO cells/ml were incubated, according to example 2, with the microcarrier according to example 1. The suspension was stirred and the cells were counted as above at different time intervals.

| Time (h) | Cells/ml |
|---|---|
| 22 | $4.6\times10^4$ |
| 64 | $1.0\times10^5$ |
| 134 | $4.4\times10^5$ |
| 186 | $5.6\times10^5$ |
| 280 | $1.4\times10^6$ |

Example 4

Autoclaved microcarriers, prepared according to example 1 but having a total capacity of 0.54 meq/g were incubated with $2\times10^5$ MRC-5 cells/ml as in Example 2 (MRC-5 is an established cell line of limited life time, originating from humanlung fibroblasts and being available from Flow Laboratories). After an initial stationary culture for 24 hours the suspension was stirred. Samples were collected after the time intervals given below and the cells were counted in the same way as given in Example 2.

| Time (h) | Cells/ml |
|---|---|
| 5 | $1.4\times10^5$ |
| 24 | $3.0\times10^5$ |
| 48 | $5.2\times10^5$ |
| 72 | $5.6\times10^5$ |

The examples 2—4 very clearly point out that microcarriers, according to the invention, result in a growth being as good as that one obtained by microcarriers previously known.

Example 5

Cultures of monkey kidney cells were washed with medium 199 (from Flow Laboratories) after 7 days in culture. Proteins were detected by counter electrophoresis.

Culturing was separately carried out on both Cytodex$^R$ 1 (Pharmacia Fine Chemicals AB) and on the surface substituted microcarrier prepared as in Example 1.

This experiment showed that, after one washing of each type of microcarrier by equal volumes of medium 199, the supernatant from the microcarrier described in Example 1 only contained 1/8 of the albumin content and 1/4 of the IgG content, which could be detected in the supernatant from Cytodex$^R$ 1.

This proves that microcarriers according to the invention adsorb considerably less proteins than the microcarriers previously known.

## Claims

1. Use of a water-swellable and water-insoluble polymeric substance which has quaternary amino groups of the formula

$$-O-CH_2-CH(OH)-CH_2-\overset{\overset{\displaystyle R_1}{|}}{\underset{\underset{\displaystyle R_2}{|}}{N^+}}-R_3$$

where $R_1$, $R_2$, and $R_3$, which are identical or different, are lower alkyl groups of 1—5 carbon atoms, or lower hydroxyalkyl groups having 2—5 carbon atoms and 1—2 hydroxy groups with at most one hydroxy group being bound to one and the same carbon atom in the hydroxyalkyl group, said quaternary amino groups being located to an outer layer of the microcarrier, which corresponds to 20—30% of the radius of the microcarrier in swollen state, as microcarrier for culturing anchorage dependent cells.

2. Use according to Claim 1 wherein the polymeric substance is cross-linked dextran.

3. Use according to Claim 1 wherein the quaternary amino groups have the formula

$$-O-CH_2CH(OH)-CH_2-N^+(CH_3)_3.$$

4. Use according to Claim 1, 2 or 3, wherein the substitution degree for the quaternary amino groups corresponds to a total charge capacity of 0.5—0.8 meq/g dry product (with chloride as a counter ion).

## Patentansprüche

1. Verwendung einer in Wasser quellbaren und in Wasser unlöslichen polymeren Substanz, die quaternäre Aminogruppen der Formel

$$-O-CH_2-CH(OH)-CH_2-\overset{\displaystyle R_1 \atop |}{\underset{| \atop \displaystyle R_2}{N^+}}-R_3$$

worin $R_1$, $R_2$ und $R_3$, die gleich oder verschieden sein können, Niedrigalkylgruppen mit 1 bis 5 Kohlenstoffatomen oder Niedrighydroxyalkylgruppen mit 2 bis 5 Kohlenstoffatomen und 1 bis 2 Hydroxygruppen, wobei höchstens eine Hydroxygruppe an eine und dasselbe Kohlenstoffatom in der Hydroxyalkylgruppe gebunden ist, sind, aufweist, wobei die genannten quaternären Aminogruppen an einer äußeren Schicht des Mikroträgers, die 20 bis 30% des Radius des Mikroträgers im gequollenen Zustand entspricht, angeordnet sind, als Mikroträger für die Kultivierung von daran verankerten und daranhängenden Zellen.

2. Verwendung nach Anspruch 1, dadurch gekennzeichnet, daß die polymere Substanz vernetztes Dextran ist.

3. Verwendung nach Anspruch 1, dadurch gekennzeichnet, daß die quaternären Aminogruppen die Formel

$$-O-CH_2-CH(OH)-CH_2-N^+(CH_3)_3$$

haben.

4. Verwendung nach Anspruch 1, 2 oder 3, dadurch gekennzeichnet, daß der Substitutionsgrad für die quaternären Aminogruppen einer Gesamtladungskapazität von 0,5 bis 0,8 mäq/g Trockenprodukt (mit Chlorid als Gegenion entspricht).

**Revendications**

1. Utilisation d'une substance polymère gonflable dans l'eau sans y être soluble, qui comprend des groupes d'ammonium quaternaire représentés par la formule ci-aprés,

$$-O-CH_2-CH(OH)-CH_2-\overset{\displaystyle R_1 \atop |}{\underset{| \atop \displaystyle R_2}{N^+}}-R_3$$

dans laquelle $R_1$, $R_2$ et $R_3$ qui peuvent être identiques ou différents, sont des radicaux alcoyles inférieurs contenant 1 à 5 atomes de carbone ou hydroxyalcoyles inférieurs ayant 2 à 5 atomes de carbone et 1 à 2 groupes hydroxy, un groupe hydroxy au plus étant lié à un et au même atome de carbone dans le groupe hydroxyalcoyle, ces groupes d'ammonium quaternaire étant situés sur une couche extérieure du microsupport, qui correspond à 20—30% du rayon du microsupport à l'état gonflé, comme microsupport pour la culture de cellules nécessitant une fixation.

2. Utilisation suivant la revendication 1, caractérisée en ce que la substance polymère est du dextrane réticulé.

3. Utilisation suivant la revendication 1, caractérisée en ce que les groupes d'ammonium quaternaire sont représentés par la formule ci-après:

$$-O-CH_2-CH(OH)-CH_2-N^+(CH_3)_3$$

4. Utilisation suivant l'une des revendications 1, 2 ou 3, caractérisée en ce que le degré de substitution pour les groupes d'ammonium quaternaire correspond à une capacité de charge totale de 0,5 à 0,8 meq/g de produit sec (avec le chlorure comme ion complémentaire).